# EUROPEAN PATENT APPLICATION

(11) **EP 1 329 517 A1**
(43) Date of publication of application: **23.07.2003**
(21) Application number: 02405033.8
(22) Date of filing: 22.01.2002
(51) Int. Cl.: C12Q 1/68

(54) **Method for the non-invasive prenatal diagnosis of chromosomal and/or genetic abnormalities**

(71) Applicant: Holzgreve, Wolfgang, Prof., 4052 Basel (CH); Hahn, Sinueh, Dr., 4410 Liestal (CH); Zimmermann, Bernard, 4031 Basel (CH)
(72) Inventor: Holzgreve, Wolfgang, Prof., 4052 Basel (CH); Hahn, Sinueh, Dr., 4410 Liestal (CH); Zimmermann, Bernard, 4031 Basel (CH)
(74) Representative: Schwander, Kuno

(57) **Abstract**

The detection of gross chromosomal abnormalities is a major focus of invasive prenatal diagnosis testing. The object of the present invention is to suggest a new method for the diagnosis of chromosomal and/or genetic abnormalities that is readily amenable to automation and high-throughput screening. This stated object of the invention is achieved by a method, characterised by the following steps:
- obtaining DNA or RNA with the genetic test locus,
- obtaining DNA or RNA of a control locus and
- detecting of the chromosomal and/or genetically determined abnormalities by Real-Time Polymerase Chain Reaction (RT-PCR) analysis, which detects specific nucleic acid amplification products as they accumulate in real-time.
In particular the method is further characterised by a multiplex RT-PCR assay in which amplification of both loci can been simultaneously monitored in the same reaction vessel, by a fluorescently labeled oligonucleotide probe, which eliminates the need for post-PCR processing. It is a preferred object of the invention to provide the method for the detection of trisomy 21 and in this way sequence specific primers for this specific analysis has been designed.

Sequence Listing

<110>	Holzgreve Wolfgang, Prof., et al
<120>	Method for diagnosis of chromosomal and/or genetic
	abnormalities
<130>	02 405 033.8-1222
<140>	EP 02 405 033.8
<141>	2002-01-22
<150>	-
<151>	-
<160>	6
<170>	Microsoft Word

<210>	1
<211>	24
<212>	RNA
<213>	Artificial Sequence
<220>	-
<221>	-
<223>	Forward-Primer

<400>	GGGAGCTGGT ACAGAAATGA CTTC	24

<210>	2
<211>	19
<212>	RNA
<213>	Artificial Sequence
<220>	-
<221>	-
<223>	Reverse-Primer

<400>	TTGCTCATTG CGCTGACAA	19

<210> 3
<211> 22
<212> RNA
<213> Artificial Sequence
<220> -
<221> -
<223> Probe wherein at the 5'-end FAM (6-carboxy-
fluorescein) and at the 3'-end TAMRA (6-carboxy-
tetramethylrhodamine) is located.

<400> AGCCATCCTT CCCGGGCCTA GG 22

<210> 4
<211> 21
<212> RNA
<213> Artificial Sequence
<220> -
<221> -
<223> Forward-Primer

<400> CCCCACACAC ATGCACTTAC C 21

<210> 5
<211> 21
<212> RNA
<213> Artificial Sequence
<220> -
<221> -
<223> Reverse-Primer

<400> CCTAGTCCCA GGGCTTTGAT T 21

<210>   6
<211>   29
<212>   RNA
<213>   Artificial Sequence
<220>   -
<221>   -
<223>   Probe wherein at the 5'-end VIC and at the 3'-end
TAMRA (6-carboxytetramethylrhodamine) is located.

<400>   AAAGAGCTAG GAAGGACAGG CAACTTGGC                    29

## Description

The detection of gross chromosomal abnormalities is a major focus of invasive prenatal diagnosis testing, of which the most common cytogenetic anomaly in live births is trisomy 21, the Down syndrome. Classically these examinations are lengthy procedures relying on karyotypic analysis of cultured amniocytes or chorionic mesenchyme. More rapid alternatives, such as fluorescence in situ hybridisation and quantitative fluorescent Polymerase Chain Reaction (PCR) on uncultured cells, are time- and labor-intensive.

Based on this knowledge it is the object of the present invention to suggest a new method for the diagnosis of chromosomal and/or genetic abnormalities that is readily amenable to automation and high-throughput screening.

This stated object of the invention is achieved by the method according to claim 1.

In particular the method according to the invention is a Real-Time PCR assay to determine chromosomal ploidy which detects specific nucleic acid amplification products as they accumulate in real-time.

The Real-Time PCR (RT-PCR) normally uses a fluorescently labeled oligonucleotide probe, which eliminates the need for post-PCR processing. It is capable of screening genetic activity within hours using a minimal amount of sample material and can detect a single molecule of DNA or RNA.

How RT-PCR Works: During polymerisation, a reporter fluorescence dye and a quencher dye are attached to a TaqMan® probe. Negligible fluorescence from the reporter dye's emission is observed once both dyes are attached to the probe. Once PCR amplification begins, DNA polymerase cleaves the probe, and the reporter dye is released from the probe. The reporter dye, which is separated from the quencher dye during every amplification cycle, generates a sequence-specific fluorescent signal. The signal increases in real time as PCR cycles continue, the fluorescence intensity increases proportionally.

RT-PCR addresses the problem of end-point analysis, which is commonly seen in traditional PCR assays. Too much amplification makes it impossible to quantitate the amount of starting nucleic acid material. RT-PCR monitors the product accumulation as the PCR amplification proceeds, allowing for higher sensitivity. Another advantage of real-time PCR is that the PCR tubes don't need to be opened until the amplification reaction is complete. This prevents contamination by PCR products and reduces the amount of "false positive" results.

As stated above the invention is providing a method for prenatal diagnosis of chromosomal and/or genetic abnormalities by using a RT-PCR system.

It has been found, surprisingly, that the RT-PCR assay can be used for the prenatal detection of Down syndrome (trisomy 21). For this purpose an adapted new technique to detect chromosomal and/or genetic abnormalities has been designed and RT-PCR and assay conditions optimised.

Therefore it is a preferred object of the invention to provide a method for the detection of trisomy 21 and in this way sequence specific primers for this specific analysis has been designed.

For the preferred object of the invention it has been established the coamplification of the genetic locus (amyloid gene) in the Down's region of chromosome 21 and of the control locus [glyceraldehyde 3-phosphate dehydrogenase (GAPDH)] on chromosome 12. The amyloid gene locus on chromosome 21 has been chosen because aberrant expression of this protein has been implicated in the physiologic lesions associated with Down syndrome. Furthermore, use of this locus should enable the detection of unbalanced Robertsonian translocations involving the Down's region of chromosome 21, which occur in about 4% of Down syndrome cases.

In the preferred embodiment of the invention the real-time PCR uses a fluorescently labeled oligonucleotide probe.

Further advantageous embodiments of the invention become evident from the dependent claims.

Advantages in the method according to the invention compared to other methods for testing chromosomal abnormalities are:
- It specifically detects chromosomal and genetic abnormalities in a very early phase of cell-development.
- It permits the analysis in a automated manner.
- The technique can be easily extended to examine the most common foetal aneuploidies on the chromosomes 13, 16, 18 and x, y.
   The present invention will now be illustrated in more detail by the following experiments, which are not meant to limit the scope of the invention. This experiments are described with reference to the drawing which shows the segregation of trisomy 21 samples from those with a normal karyotype by multiplex RT-PCR-assay.

### Real-Time PCR - theoretical basis:

RT-PCR is the most sensitive technique to determine the presence or absence of DNA or RNA templates or to quantify the level of gene expression. Furthermore RT-PCR allows cloning of rare messages without the necessity of construction cDNA libraries.

Real-time quantitative PCR monitors the degradation of a sequence-specific, dual-labeled fluorescent probe after each cycle of PCR amplification. During the extension phase, the 5'-exonuclease activity of Taq DNA polymerase cleaves the probe, separating the 5'-reporter fluorescent dye from the 3'-quencher fluorescent dye, resulting in an increase in the emission spectra of the reporter fluorescent dye. After subtraction of the background fluorescence, calculated during the first, for example 15 amplification cycles, the measured fluorescence is graphed as an amplification plot. Each reaction is characterised by a value, Ct, defined as the fractional number of cycles at which the reporter fluorescent emission reaches a fixed threshold level in the exponential region of the amplification plot. The Ct value is correlated to input target DNA and/or mRNA amount: a larger starting quantity of DNA and/or mRNA target results in a lower number of PCR cycles required for the reporter fluorescent emission to reach the threshold, and therefore a lower Ct value. Thus, the method is not based on the measurement of the total amount of amplified product after a fixed number of cycles, as in conventional PCR, and does not require post-PCR processing of the product.

### Primers and Probes:

Primers and probes for trisomy 21 (i.e. the amyloid gene on chromosome 21) are as follows:
Forward-Primer:
   5'-GGG AGC TGG TAC AGA AAT GAC TTC-3'
Reverse-Primer:
   5'-TTG CTC ATT GCG CTG ACA A-3'
Probe:
   5'-(FAM) AGC CAT CCT TCC CGG GCC TAG G (TAMRA)-3',
   wherein
   FAM is 6-carboxyfluorescein and
   TAMRA is 6-carboxytetramethylrhodamine.

Primers and probes of the control locus GAPDH are as follows:
Forward-Primer:
   5'-CCC CAC ACA CAT GCA CTT ACC-3'
Reverse-Primer:
   5'-CCT AGT CCC AGG GCT TTG ATT-3'
Probe:
   5'-(VIC) AAA GAG CTA GGA AGG ACA GGC AAC TTG GC (TAMRA)-3'

The primers and probes has been designed in order to display minimal internal structure (i.e., primer-dimer formation) and similar melting temperatures. Optimal concentrations for the designed primers and probes has been determined as the lowest ones giving the highest fluorescence levels and the lowest Ct values.

### PCR Amplification and Analysis:

For the experiments a multiplex RT-PCR assay was used in which amplification of both loci can been simultaneously monitored in the same reaction vessel. In this way, one could be sure that any alterations were not attributable to well-to-well variation. Further, the PCR reactions were performed and monitored using a Sequence Detection System of Perkin-Elmer Applied Biosystem.

In the TaqMan® analysis, 25 µl reaction volumes containing
- 2µl of the extracted DNA,
- 300 nmol/l each of the primers,
- 150 nmol/l each of the dual-labeled TaqMan® probes, and
- further components supplied in the TaqMan® Universal PCR Master Mix (Perkin-Elmer), corresponding to 3,5 mmol/l MgCl₂, 100 µmol/l dNTPs, 0,025 U/µl AmpliTaq Gold, and
   0,01 U/µl Amp Erase,
   were used.

Cycling conditions were as follows: incubation for 2 min at 50°C to permit Amp Erase activity and for 10 min at 95°C for AmpliTaq Gold activation and DNA denaturation, followed by 40 cycles of 1 min at 60°C and 15 s at 95°C.

For each sample, the Ct value of each target sequence was subtracted to the Ct value of the reference gene (GAPDH), to derive ΔCt.

In proof-of-principle experiments DNA extracted from amniocyte cultures obtained from 10 trisomy 21 foetuses were used. As controls DNA samples obtained from 11 apparently healthy individuals were used. The samples included peripheral blood as well as amniocyte cultures.

For the experiments DNA was extracted from 400 µl samples, using the QlAamp DNA Blood Mini Kit according to the manufacturer's recommendations (Qiagen). Further, several dilutions of the DNA samples were included in order to concern that the assay is applicable over a broad range of DNA concentrations.

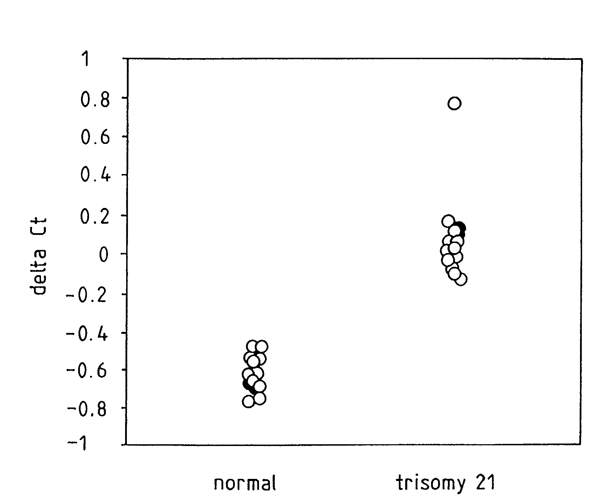

The results of these small series of experiments are illustrated in figure 1 and show that the ratio of the two loci in the samples, as determined by the difference in threshold cycle value (ΔCt), distinguished trisomy 21 from karyotypically normal tissue. Figure 1 shows a clear segregation of trisomy 21 samples from those with a normal karyotype. Differences in threshold cycle numbers (ΔCt) greater than - 0,2 are indicative of a trisomy 21 karyotype, whereas differences less than - 0,4 indicate a normal karyotype.

Furthermore, inventors ascertained that clear segregation of karyotypically normal and trisomic samples was possible over a wide sample concentration range, in that one still observed optimal results when 10-fold diluted DNA samples and DNA concentrations as low as 10 ng/µl were used.

In order to achieve the high degrees of accuracy required for prenatal diagnosis, it was decided to include some additional safety precautions. These meant that the following two conditions had to be met to permit a reliable and robust assay: firstly that the concentration and purity of the DNA sample were such that an efficient amplification of both loci examined occurs. Secondly, that both loci were amplified with equal efficiency over the entire exponential phase of the RT-PCR. For this purpose two examination assays have been carried out. For the first step, the amplitude of the amplification curve (ΔR_{N}) was examined. Because this parameter is indicative of amplification efficiency and template concentration, it can be considered a rather basic endpoint measurement for quality surveillance. In the experiments cited above one arbitrarily selected a ΔR_{N} cut-off value of 0,7. It is possible that a another cut-off value may be obtained in a other, i.e. larger, series of experiments. For the second step, which was to ensure that both loci had been amplified with equal efficiency, one examined their ratio at three separate points (bottom, middle, top) along the exponential phase of the amplification reaction. Samples in which a deviation was found were disregarded.

Using this approach, it was possible to correctly determine the ploidy in 9 of 11 cases with normal karyotype. The analysis included six diluted samples in which the ploidy was also identified correctly. In the 10 samples from trisomy 21 foetuses, it was possible to determine the ploidy correctly in 9 samples and in 10 separate dilutions. In those cases where one could not reliably determine foetal ploidy the inventors were alerted to incorrect amplification either by a ΔR_{N} value < 0,7 or by an inconsistency in the ratio of the two loci examined at the three thresholds selected over the exponential phase of the amplification. In one case of trisomy 21, an outliner was recorded with a ΔCt of almost 0,8. This sample was almost 3 years old. Interestingly, in two dilutions of this extraction, one obtained the expected ΔCt values for trisomy 21.

The results of the above described experiments show that RT-PCR technology can be used for the rapid determination of trisomy 21. Further the method according to the invention can be easily extended to examine the most common foetal aneuploidies on the chromosomes 13, 16, 18, X and Y. It is also noted that the test can be used to test sperm for possible paternally inherited aneuploidies. This is a frequent constellation in couples who have fertility problem.

Because RT-PCR permits the analysis of numerous samples in an automated manner, the method according to the invention may be more suited to this task than current molecular or cellular cytogenetic methods because these are considerably more time- and labor-intensive.

In a large-scale setting, the method according to invention may also compare favourably with a fluorescent PCR-based approach or fluorescence in situ hybridisation analysis regarding speed and price.

## Claims

1. Method for diagnosis of chromosomal and/or genetic abnormalities, **characterised by** the following steps:
- obtaining DNA or RNA with the genetic test locus,
- obtaining DNA or RNA of a control locus and
- detecting of the chromosomal and/or genetically determined abnormalities by Real-Time Polymerase Chain Reaction (RT-PCR) analysis, which detects specific nucleic acid amplification products as they accumulate in real-time.

2. Method according to claim 1, **characterised in that** the following two conditions will be met in order to permit a reliable and robust assay:
- firstly that the concentration and purity of the DNA sample is such that an efficient amplification of both loci examined occurs;
- secondly, that both loci will be amplified with equal efficiency over the entire exponential phase of the RT-PCR.

3. Method according to claim 1 or 2, **characterised in that** a sequence on the chromosome to be examined is amplified and quantified in relation to a control sequence of another chromosome.

4. Method according to one of the claims 1 to 3 for prenatal diagnosis of trisomy 21.

5. Method according to claim 4, **characterised in that** the genetic test locus is the amyloid gene in the Down's region of chromosome 21 and that the control locus is the gene of glyceraldehyde 3-phosphate dehydrogenase (GAPDH) on chromosome 12,

6. Method according to one of the claims 4 or 5 for amplification of the amyloid gene, **characterised in that** the forward primer comprises the following sequence
5'-GGG AGC TGG TAC AGA AAT GAC TTC-3'.

7. Method according to one of the claims 4 to 6 for amplification of the amyloid gene, **characterised in that** the reverse primer comprises the following sequence
5'-TTG CTC ATT GCG CTG ACA A-3'.

8. Method according to one of the claims 4 to 7 for amplification of the amyloid gene, **characterised in that** the RT-PCR uses a fluorescently labeled oligonucleotide probe comprising the following sequence
5'-(FAM) AGC CAT CCT TCC CGG GCC TAG G (TAMRA)-3',
wherein
FAM is 6-carboxyfluorescein and
TAMRA is 6-carboxytetramethylrhodamine.

9. Method according to one of the claims 4 to 8 for amplification of the control locus GAPDH, **characterised in that**
the forward primer comprises the sequence
5'-CCC CAC ACA CAT GCA CTT ACC-3';
the reverse primer comprises the sequence
5'-CCT AGT CCC AGG GCT TTG ATT-3';
the RT-PCR uses a fluorescently labeled oligonucleotide probe comprising the sequence
5'-(VIC) AAA GAG CTA GGA AGG ACA GGC AAC TTG GC (TAMRA)-3'.

10. Method according one of the claims 1 to 9, **characterised by** a multiplex RT-PCR assay in which amplification of both loci can been simultaneously monitored in the same reaction vessel.
